# EUROPEAN PATENT SPECIFICATION

(11) **EP 1 408 024 B1**
(45) Date of publication and mention of the grant of the patent: **20.10.2010**
(21) Application number: 02746039.3
(22) Date of filing: 15.07.2002
(51) Int. Cl.: C07C 43/23, C07C 41/40, C07C 41/26

(54) **METHOD OF CRYSTALLIZING REDUCED COENZYME Q sb 10 /sb FROM AQUEOUS SOLUTION**
VERFAHREN ZUM KRISTALLISIEREN VON REDUZIERTEM COENZYM Q10 AUS WÄSSRIGER LÖSUNG
PROCEDE DE CRISTALLISATION D'UNE COENZYME Q10 REDUITE A PARTIR D'UNE SOLUTION AQUEUSE

(30) Priority: 13.07.2001 JP 2001214477; 17.04.2002 JP 2002114874
(43) Date of publication of application: 14.04.2004
(73) Proprietor: KANEKA CORPORATION, Osaka-shi, Osaka 530-8288 (JP)
(72) Inventor: UEDA, Takahiro, Kobe-shi, Hyogo 655-0872 (JP); KITAMURA, Shiro, Akashi-shi, Hyogo 673-0882 (JP); UEDA, Yasuyoshi, Himeji-shi, Hyogo 671-1227 (JP)
(74) Representative: HOFFMANN EITLE
(86) International application number: PCT/JP2002/007146
(87) International publication number: WO 2003/006411

(56) References cited:
- GB-A- 947 643
- JP-A- 52 072 884
- JP-A- 56 092 238

## Description

### TECHNICAL FIELD

The present invention relates to a method of crystallizing reduced coenzyme Q₁₀. Reduced coenzyme Q₁₀ shows a higher level of oral absorbability as compared with oxidized coenzyme Q₁₀ and is a compound useful as an ingredient in good foods, functional nutritive foods, specific health foods, nutritional supplements, nutrients, drinks, feeds, animal drugs, cosmetics, medicines, remedies, preventive drugs, etc.

### BACKGROUND ART

It is known that reduced coenzyme Q₁₀ can be prepared by producing coenzyme Q₁₀ in the conventional manner, for example by synthesis, fermentation, or extraction fromnatural products, and concentrating a reduced coenzyme Q₁₀-containing eluate fraction resulting from chromatography (JP-A-10-109933). On that occasion, as described in the above-cited publication, the chromatographic concentrationmaybe carried out after reduction of oxidized coenzyme Q₁₀ contained in the reduced coenzyme Q₁₀ with a reducing agent such as sodium borohydride or sodium dithionite (sodium hyposulfite), or reduced coenzyme Q₁₀ may be prepared by reacting the reducing agent mentioned above with an existing highly pure grade of coenzyme Q₁₀ (oxidized form) .

However, the thus-obtained reduced coenzyme Q₁₀ is not always easy to be crystallized preferably but tends to occur as a low-purity crystalline, semisolid, or oily product containing such impurities as oxidized coenzyme Q₁₀. Moreover, even when crystallization could be achieved somehow, some troubles are occurred due to its poor slurry properties, etc. For example, poor slurry fluidity causes stirring trouble or difficulty in brushing away from a crystallization container, poor filterability, and the crystallization yield which is not always high.

In addition, it is very uneconomical to use a large amount of organic solvents in crystallization. Moreover, these organic solvents are brought into products and tend to give unfavorable characteristics to the products which humans take in. In order to decrease the residual amount of the organic solvent in products to a level lower than the trace amount, too much time and expensive manufacture equipment are necessary for removal of the organic solvent, drying, etc.

GB 947, 643 discloses that reduced coenzyme Q₁₀ was prepared from oxidized coenzyme Q₁₀ and it may be recrystallized from a mixture of alcohol and petroleum ether.

### SUMMARY OF THE INVENTION

In view of the above, the present invention has its object for providing an excellent crystallization method for obtaining the reduced coenzyme Q₁₀ crystal suitable for the industrial-scale production.

As a result of intensive investigations, the present inventors found that the solubility and fluidity of the reduced coenzyme Q₁₀ may be preferably controlled by using water, and that the high-quality reduced coenzyme Q₁₀ crystal maybe obtained by crystallizing the reduced coenzyme Q₁₀ in an aqueous solution for improving the slurry property, yield, etc., and thereby completed the present invention.

That is, the present invention relates to a method for crystallizing the reduced coenzyme Q₁₀ which comprises a crystallization of the reduced coenzyme Q₁₀ in an aqueous solution.

### DETAILED DESCRIPTION OF THE INVENTION

Hereinafter, the present invention is described in detail.

The reduced coenzyme Q₁₀ which can be used in the present invention can be obtained in the conventional manner, for example by synthesis, fermentation, or extraction fromnatural products. Preferably, it can be obtained by reducing oxidized coenzymes Q₁₀ such as an existing high-purity coenzyme Q₁₀, or a mixture of the oxidized coenzyme Q₁₀ and the reduced coenzyme Q₁₀ using a common reducing agent.

Firstly, a method for reducing the oxidized coenzyme Q₁₀ is explained. Since reduced coenzyme Q₁₀ is readily oxidized by molecular oxygen to give oxidized coenzyme Q₁₀ as a byproduct, a solvent high in protective effect against oxidation is preferably used as the solvent in the step of reduction. Preferably used as such solvent is at least one species selected from among hydrocarbons, fatty acid esters, ethers, and nitriles. Hydrocarbons are most preferred.

The hydrocarbons are not particularly restricted, but there may be mentioned, for example, aliphatic hydrocarbons, aromatic hydrocarbons, halogenated hydrocarbons, etc. Preferred are aliphatic hydrocarbons and aromatic hydrocarbons, and more preferred are aliphatic hydrocarbons.

The aliphatic hydrocarbons are not particularly restricted, and may be cyclic or acyclic, or saturated or unsaturated. However, generally they contain 3 to 20 carbon atoms, and preferably 5 to 12 carbon atoms.

As specific examples, there may be mentioned, for example, propane, butane, isobutane, pentane, 2-methylbutane, cyclopentane, 2-pentene, hexane, 2-methylpentane, 2,2-dimethylbutane, 2,3-dimethylbutane, methylcyclopentane, cyclohexane, 1-hexene, cyclohexene, heptane, 2-methylhexane, 3-methylhexane, 2,3-dimethylpentane, 2,4-dimethylpentane, methylcyclohexane, 1-heptene, octane, 2,2,3-trimethylpentane, isooctane, ethylcyclohexane, 1-octene, nonane, 2,2,5-trimethylhexane, 1-nonene, decane, 1-decene, p-menthane, undecane, dodecane, etc.

Among them, saturated aliphatic hydrocarbons having 5 to 8 carbon atoms are more preferred, and preferably used are pentane, 2-methylbutane and cyclopentane, which have 5 carbon atoms (referred to as "pentanes"); hexane, 2-methylpentane, 2,2-dimethylbutane, 2,3-dimethylbutane, methylcyclopentane, cyclohexane, which have 6 carbon atoms (referred to as "hexanes"); heptane, 2-methylhexane, 3-methylhexane, 2,3-dimethylpentane, 2,4-dimethylpentane, methylcyclohexane, which have 7 carbon atoms (referred to as "heptanes"); octane, 2,2,3-trimethylpentane, isooctane, ethylcyclohexane, which have 8 carbon atoms (referred to as octanes); and a mixture of these. In particular, the above heptanes are particularly preferred since they have a tendency to show a very high protection effect against oxidization, and heptane is most preferred.

The aromatic hydrocarbons are not particularly restricted, but generally they contain 6 to 20 carbon atoms, preferably 6 to 12 carbon atoms, and more preferably 7 to 10 carbon atoms. As specific examples, there may be mentioned, for example, benzene, toluene, xylene, o-xylene, m-xylene, p-xylene, ethylbenzene, cumene, mesitylene, tetralin, butylbenzene, p-cymene, cyclohexylbenzene, diethylbenzene, pentylbenzene, dipentylbenzene, dodecylbenzene, styrene, etc. Preferred are toluene, xylene, o-xylene, m-xylene, p-xylene, ethylbenzene, cumene, mesitylene, tetralin, butylbenzene, p-cymene, cyclohexylbenzene, diethylbenzene and pentylbenzene. More preferred are toluene, xylene, o-xylene, m-xylene, p-xylene, cumene and tetralin, and most preferred is cumene.

The halogenated hydrocarbons are not particularly restricted, and may be cyclic or acyclic, or saturated or unsaturated. However, acyclic halogenated hydrocarbons are preferably used. More preferred are chlorinated hydrocarbons and fluorinated hydrocarbons, and chlorinated hydrocarbons are still more preferred. Additionally, ones containing 1 to 6 carbon atoms, preferably 1 to 4 carbon atoms, and more preferably 1 to 2 carbon atoms are used.

As specific examples, for example, there may be mentioned dichloromethane, chloroform, carbon tetrachloride, 1,1-dichloroethane, 1,2-dichloroethane, 1,1,1-trichloroethane, 1,1,2-trichloroethane, 1,1,1,2-tetrachloroethane, 1,1,2,2-tetrachloroethane, pentachloroethane, hexachloroethane, 1,1-dichloroethylene, 1,2-dichloroethylene, trichloroethylene, tetrachloroethylene, 1,2-dichloropropane, 1,2,3-trichloropropane, chlorobenzene, 1,1,1,2-tetrafluoroethane, etc.

Preferred are dichloromethane, chloroform, carbon tetrachloride, 1,1-dichloroethane, 1,2-dichloroethane, 1,1,1-trichloroethane, 1,1,2-trichloroethane, 1,1-dichloroethylene, 1,2-dichloroethylene, trichloroethylene, chlorobenzene and 1,1,1,2-tetrafluoroethane. More preferred are dichloromethane, chloroform, 1,2-dichloroethylene, trichloroethylene, chlorobenzene and 1,1,1,2-tetrafluoroethane.

The fatty acid esters are not particularly restricted, but there may be mentioned, for example, propionates, acetates, formates, etc. Preferred are acetates and formates, and more preferred are acetates. Ester functional groups thereof are not particularly restricted, but alkyl esters having 1 to 8 carbon atoms, aralkyl esters having 1 to 8 carbon atoms are used, preferred are alkyl esters having 1 to 6 carbon atoms, and more preferred alkyl esters having 1 to 4 carbon atoms.

As the propionates, there may be mentioned, for example, methyl propionate, ethyl propionate, butyl propionate, isopentyl propionate, etc. Preferred is ethyl propionate.

As the acetates, there maybe mentioned, for example, methyl acetate, ethyl acetate, propyl acetate, isopropyl acetate, butyl acetate, isobutyl acetate, sec-butyl acetate, pentyl acetate, isopentyl acetate, sec-hexyl acetate, cyclohexyl acetate, benzyl acetate, etc. Preferred are methyl acetate, ethyl acetate, propyl acetate, isopropyl acetate, butyl acetate, isobutyl acetate, sec-butyl acetate, pentyl acetate, isopentyl acetate, sec-hexyl acetate and cyclohexyl acetate. More preferred are methyl acetate, ethyl acetate, propyl acetate, isopropyl acetate, butyl acetate and isobutyl acetate. Most preferred is ethyl acetate.

As the formates, there may be mentioned, for example, methyl formate, ethyl formate, propyl formate, isopropyl formate, butyl formate, isobutyl formate, sec-butyl formate, pentyl formate, etc. Preferred are methyl formate, ethyl formate, propyl formate, butyl formate, isobutyl formate and pentyl formate, and most preferred is ethyl formate.

The ethers are not particularly restricted, and may be cyclic or acyclic, or saturated or unsaturated. But saturated ones are preferably used. Generally, ones containing 3 to 20 carbon atoms, and preferably 4 to 12 carbon atoms and more preferably 4 to 8 carbon atoms are used.

As specific examples, there may be mentioned, for example, diethyl ether, methyl tert-butyl ether, dipropyl ether, diisopropyl ether, dibutyl ether, dihexyl ether, ethyl vinyl ether, butyl vinyl ether, anisol, phenetole, butyl phenyl ether, methoxytoluene, dioxane, furan, 2-methylfuran, tetrahydrofuran, tetrahydropyran, ethylene glycol dimethyl ether, ethylene glycol diethyl ether, ethylene glycol dibutyl ether, ethylene glycol monomethyl ether, ethylene glycol monoethyl ether, ethylene glycol monobutyl ether, etc.

Preferred are diethyl ether, methyl tert-butyl ether, dipropyl ether, diisopropyl ether, dibutyl ether, dihexyl ether, anisol, phenetole, butyl phenyl ether, methoxytoluene, dioxane, 2-methylfuran, tetrahydrofuran, tetrahydropyran, ethylene glycol dimethyl ether, ethylene glycol diethyl ether, ethylene glycol dibutyl ether, ethylene glycol monomethyl ether and ethylene glycol monoethyl ether. More preferred are diethyl ether, methyl tert-butyl ether, anisol, dioxane, tetrahydrofuran, ethylene glycol monomethyl ether and ethylene glycol monoethyl ether. More preferred are diethyl ether, methyl tert-butyl ether, anisol, etc., and most preferred is methyl tert-butyl ether.

The nitriles are not particularly restricted, and may be cyclic or acyclic, or saturated or unsaturated. However, saturated ones are preferably used. Generally, ones containing 2 to 20 carbon atoms, preferably 2 to 12 carbon atoms, and more preferably 2 to 8 carbon atoms are used.

As specific examples, there may be mentioned, for example, acetonitrile, propiononitrile, malononitrile, butyronitrile, isobutyronitrile, succinonitrile, valeronitrile, glutaronitrile, hexanenitrile, heptylcyanide, octylcyanide, undecanenitrile, dodecanenitrile, tridecanenitrile, pentadecanenitrile, stearonitrile, chloroacetonitrile, bromoacetonitrile, chloropropiononitrile, bromopropiononitrile, methoxyacetonitrile, methyl cyanoacetate, ethyl cyanoacetate, tolunitrile, benzonitrile, chlorobenzonitrile, bromobenzonitrile, cyanobenzoic acid, nitrobenzonitrile, anisonitrile, phthalonitrile, bromotolunitrile, methyl cyanobenzoate, methoxybenzonitrile, acetylbenzonitrile, naphthonitrile, biphenylcarbonitrile, phenylpropiononitrile, phenylbutyronitrile, methylphenylacetonitrile, diphenylacetonitrile, naphthylacetonitrile, nitrophenylacetonitrile, chlorobenzylcyanide, cyclopropanecarbonitrile, cyclohexanecarbonitrile, cycloheptanecarbonitrile, phenylcyclohexanecarbonitrile, tolylcyclohexanecarbonitrile, etc.

Preferred are acetonitrile, propiononitrile, butyronitrile, isobutyronitrile, succinonitrile, valeronitrile, chloropropiononitrile, methyl cyanoacetate, ethyl cyanoacetate, tolunitrile and benzonitrile. More preferred are acetonitrile, propiononitrile, butyronitrile and isobutyronitrile, and most preferred is acetonitrile.

In selecting the solvent to be used from among the solvents mentioned above, such properties as boiling point and viscosity are preferably taken into consideration; for example, the solvent should have a boiling point which allows appropriate warming for increasing the solubility and facilitates a solvent removal from wet masses by drying and solvent recovery from crystallization filtrates (about 30 to 150°C at 1 atm), a melting point such that solidification hardly occurs in handling at room temperature as well as upon cooling to room temperature or below (not higher than about 20°C, preferably not higher than about 10°C, still more preferably not higher than about 0°C), and a low viscosity (not higher than about 10 cp at 20°C). From the industrial operation viewpoint, a solvent which is scarcely volatile at ordinary temperature is preferred; for example, one having a boiling point of not lower than about 80°C is preferred, and one having a boiling point of not lower than about 90°C is more preferred.

Among the solvents in the reduction reaction mentioned above, a solvent having low compatibility with water is particularly preferred. The solvent in the reduction reaction promotes purifying and obtaining a reduced coenzyme Q₁₀ efficiently, by extracting the reducing agent to be described below and/or impurities from the reducing agent and removing the same. As such solvents having low compatibility with water, there may be mentioned, for example, the above-mentioned hydrocarbons and fatty acid esters among the solvents mentioned above. Reduced coenzyme Q₁₀, when in a dissolved state, tends to become more resistant to oxidation as the concentration thereof increases. Reduced coenzyme Q₁₀ is highly soluble in the solvents mentioned above and, in this respect, too, the above solvents are suitable for the protection from oxidation. The concentration of reduced coenzyme Q₁₀ which is preferred from the viewpoint of protection thereof from oxidation may vary depending on the solvent species, among others, hence cannot be absolutely specified. Generally, however, the concentration of reduced coenzyme Q₁₀ in the above solvents is generally not lower than 1 w/w%, preferably not lower than 2 w/w%. The upper limit is not particularly restricted but, from the practical operability viewpoint, it is 400 w/w% or below, preferably 200 w/w% or below, more preferably 100 w/w% or below, still more preferably 50 w/w% or below.

Thus, when such a solvent as mentioned above is used, it is possible to minimize the undesirable oxygen-involving side reaction via the step of reduction reaction.

Additionally, the reduced coenzyme Q₁₀ used for crystallization can be also obtained by reducing oily oxidized coenzyme Q₁₀ in an aqueous solution. In this method, the reduced coenzyme Q₁₀ can be synthesized without using an organic solvent, additional operations such as extraction to an organic phase, concentration, etc. are not required, the operation time can be shortened and subgeneration of the oxidized coenzyme Q₁₀ can be minimized.

The reduction reaction can be carried out, in the above solvent, using, as a reducing agent, a metal hydride compound, iron (metallic iron or iron in a salt form), zinc (metallic zinc) dithionous acid or a salt thereof, or an ascorbic acid or a related compound, for instance.

The metal hydride compound is not particularly restricted but includes, among others, sodium borohydride, lithium aluminum hydride, etc. The amount to be used of the metal hydride compound may vary depending on the species thereof, hence cannot be absolutely specified. Generally, however, the reduction can be favorably carried out by using it in an amount of 1 to 3 times the theoretical hydrogen equivalent.

The reduction using iron or zinc is generally carried out using an acid. The acid to be used is not particularly restricted but includes, among others, fatty acids such as acetic acid, sulfonic acids such as methanesulfonic acid, inorganic acids such as hydrochloric acid and sulfuric acid, etc. Inorganic acids are preferred, and sulfuric acid is more preferred.

The amount of iron to be used is not particularly restricted but, for example, an amount of about 1/5 by weight or larger basedon the charged weight of oxidized coenzyme Q₁₀ is appropriate for carrying out the reaction. The upper limit is not particularly restricted but, from the economical viewpoint, it is about twice the weight of the above charged weight or lower. Iron may be used not only in the form of metallic iron but also in the form of a salt, for example iron(II) sulfate, etc.

The amount of zinc to be used is not particularly restricted but, for example, an amount of about 1/10 by weight or larger based on the charged weight of oxidized coenzyme Q₁₀ is appropriate for carrying out the reaction. The upper limit is not particularly restricted but, from the economic viewpoint, it is about twice the weight of the above charged weight or lower.

The dithionous acid or a salt thereof is not particularly restricted but a salt form of dithionous acid is generally used. The salt of dithionous acid is not particularly restricted but includes, as preferred species, alkali metal salts, alkaline earth metal salts, ammonium salt and the like. Alkali metal salts such as the lithium salt, sodium salt, and potassium salt are more preferred, and the sodium salt is most preferred. The amount to be used of the dithionous acid or salt is not particularly restricted but it is generally not smaller than about 1/5 by weight, preferably not smaller than about 2/5 by weight, and more preferably not smaller than about 3/5 by weight, based on the charged weight of oxidized coenzyme Q₁₀. Larger amounts may be used without causing any particularly trouble. From the economical viewpoint, however, the amount to be employed is not larger than about twice the weight of the above-mentioned charged weight, preferably not larger than the charged weight. Thus, the reaction can be more favorably carried out with employing an amount within the range of about 2/5 by weight of the above-mentioned charge to a weight roughly equal to that of the charged weight.

The ascorbic acid or a related compound are not particularly restricted, and include, for example, not only ascorbic acid, but also rhamno-ascorbic acid, arabo-ascorbic acid, gluco-ascorbic acid, fuco-ascorbic acid, glucohepto-ascorbicacid, xylo-ascorbicacid, galacto-ascorbic acid, gulo-ascorbic acid, allo-ascorbic acid, erythro-ascorbic acid, 6-desoxyascorbic acid, and the like ascorbic acid-related compounds, and may be ester forms or salts of these. Furthermore, these may be L-form, D-form or racemic form. More specifically, there may be mentioned, for example, L-ascorbic acid, L-ascorbyl palmitate, L-ascorbyl stearate, D-arabo-ascorbicacid, etc. In producing the reduced coenzyme Q₁₀, any of the above-mentioned ascorbic acid or related compounds may be suitably used. However, the water-soluble ones are suitably used in particular among the above-mentioned ascorbic acid or related compounds in view of separatability with the generated reduced coenzyme Q₁₀, etc. And most preferred is a free form of L-ascorbic acid, D-arabo-ascorbic acid, and the like in view of the ready availability, price, etc.

The amount to be used of the ascorbic acid or related compounds mentioned above is not particularly restricted but may be an amount effective in converting oxidized coenzyme Q₁₀ to reduced coenzyme Q₁₀. Generally it is not smaller than 1 mole, preferably not smaller than 1.2 moles, per mole of oxidized coenzyme Q₁₀. The upper limit is not particularly restricted but, from the economical viewpoint, it is generally not higher than 10 moles, preferably not higher than 5 moles, and more preferably not higher than 3 moles, per mole of the oxidized coenzyme Q₁₀.

Among the reducing agent species mentioned above, zinc, dithionous thereof, and ascorbic acid or related compounds are preferred from the viewpoint of reducing ability, yield and/or quality, among others. In particular, dithionous acid or salts thereof (specifically dithionous acidsalts) and ascorbic acid or related compounds (particularly, free-form or salt thereof) are preferred from a viewpoint that they bring the reducing agent or impurities derived from the reducing agent into the reduced coenzyme Q₁₀ crystal in only the trace amount or lower.

In carrying out the reduction reaction, an alcohol and/or water are/is suitably used singly or in combination, as to be mentioned below. Water is preferred in particular when iron, zinc, or dithionous acid or a salt thereof is used as the reducing agent. When a metal hydride compound or an ascorbic acid or a related compound is used as the reducing agent, an alcohol can be used in combination. The combined use of water and an alcohol exhibits the characteristics of both water and the alcohol and contributes to improvements in reaction rate and yield, among others.

In the following, a preferred method of reduction is described in detail.

The reduction using dithionous acid or a salt thereof is preferably carried out using water in combination, namely in a mixed solvent system composed of at least one organic solvent selected from among the above-mentioned hydrocarbons, fatty acid esters, ethers, and nitriles, with water. On that occasion, the reaction is preferably carried out generally at a pH of not higher than 7, preferably at pH 3 to 7, more preferably at pH 3 to 6, from the viewpoint of yield, etc. The pH can be adjusted using an acid (e.g. an inorganic acid such as hydrochloric acid or sulfuric acid) or a base (e.g. an alkali metal hydroxide such as sodium hydroxide).

In the reduction using dithionous acid or a salt thereof, the amount of water is not particularly restricted but may be an amount of water such that an appropriate amount of the reducing agent, namely dithionous acid or a salt thereof, can be dissolved therein. Thus, for example, it is advisable that the amount of the dithionous acid or a salt be adjusted generally to not more than 30 w/w%, and preferably not more than 20 w/w%, relative to the weight of water. From the productivity viewpoint, among others, it is advisable that the amount be adjusted generally to not less than 1 w/w%, preferably not less than 5 w/w%, and more preferably not less than 10 w/w%.

The reduction using the ascorbic acid or a related compound mentioned above can be carried out using a solvent especially highly miscible with water as selected from among the above-mentioned hydrocarbons, fatty acid esters, ethers, and nitriles, in particular ethers and nitriles, which are highly miscible with water, and more specifically tetrahydrofuran, dioxane, acetonitrile or the like. It is particularly preferred to use the alcohols and/or ketones to be mentioned below (preferably alcohols and/or ketones, which are highly miscible with water (in particular, monohydric or dihydric alcohols (preferably monohydric ones) having 1 to 5 carbon atoms, preferably 1 to 4 carbon atoms, more preferably 1 to 3 carbon atoms, and/or, ketones such as acetone, methyl ethyl ketone or the like)). Namely, in the reduction using the ascorbic acid or a related compound, it is preferable to use,alcohols and/or water-soluble organic solvents (the above ethers, nitriles, ketones and the like, which are highly miscible with water, for example).

Furthermore, from the viewpoint of reaction promotion (e.g. reaction temperature lowering or reaction time shortening) in the production of reduced coenzyme Q₁₀, it is also possible to carry out the reduction using the ascorbic acid or a related compound in the presence of an additive having a reaction promoting effect, such as a basic substance or a hydrogensulfite.

The basic compound is not particularly restricted but may be either an inorganic compound or an organic compound. The inorganic compound is not particularly restricted but includes, among others, the hydroxides, carbonates and hydrogencarbonates of metals (preferably alkali metals, alkaline earth metals, and the like), ammonia, etc. As typical examples thereof, there maybe mentioned alkali metal hydroxides such as sodium hydroxide, alkali metal carbonates such as sodium carbonate, alkali metal hydrogencarbonates such as sodium hydrogencarbonate, and alkaline earth metal carbonates such as magnesium carbonate. The organic compound is not particularly restricted but includes, among others, amines such as triethylamine, etc. Among the basic substances specifically mentioned above, weakly basic substances (weak bases or weak alkalis) such as the carbonates and hydrogencarbonates of metals (preferably alkali metals, alkaline earth metals, etc.), ammonia, and like inorganic compounds; amines such as triethylamine, and like organic compounds are preferably used. More preferred are the weakly basic inorganic compounds mentioned above.

Preferred as the hydrogensulfite are, for example, alkali metal hydrogensulfites such as sodium hydrogensulfite, etc.

The amount of the additive mentioned above is not particularly restricted but may be such that the reaction promoting effect of the additive can be produced to a desired extent (effective amount). From the economical viewpoint, however, the amount is generally not more than 20 moles, preferably not more than 10 moles, more preferably not more than 5 moles, and still more preferably not more than 2 moles, per mole of the ascorbic acid or a related compound. The lower limit is not particularly restricted but, generally, it is not less than 0.01 moles, preferably not less than 0.05 moles, more preferably not less than 0.1 moles, and still more preferably not less than 0.2 moles, per mole of the ascorbic acid or a related compound.

The reduction reaction is preferably carried out under forced flowing. The power required for stirring to cause such flowing per unit volume is generally not less than about 0.01 kW/m³, preferably not less than about 0.1 kW/m³, and more preferably not less than about 0.3 kW/m³. The above forced flowing is generally caused by the turning of a stirring blade (s). The use of a stirring blade(s) is not always necessary if the above flowing can be otherwise obtained. For example a method based on liquid circulation may be utilized.

The reduction temperature may vary depending on the reducing agent species and/or amount, hence cannot be absolutely specified. In the reduction using dithionous acid or a salt thereof, for instance, the reduction is generally carried out at 100°C or below, preferably at 80°C or below, more preferably at 60°C or below. The lower limit is the solidification temperature of the system. Thus, the reduction can be favorably carried out generally at about 0 to 100°C, preferably at about 0 to 80°C, more preferably at about 0 to 60°C. In the reduction using an ascorbic acid or a related compound, the reduction is carried out generally at 30°C or higher, preferably at 40°C or higher, more preferably at 50°C or higher. The upper limit is the boiling point of the system. Thus, the reduction can be favorably carried out generally at about 30 to 150°C, preferably about 40 to 120°C, more preferably at about 50 to 100°C. The reduction of the oily oxidized coenzyme Q₁₀ is generally carried out at 45° C or more, preferably at 48° C or more, and more preferably at 50°C or more, although it is dependent on the purity, etc., then the oily reduced coenzyme Q₁₀ can be obtained.

The reaction concentration is not particularly restricted but the weight of oxidized coenzyme Q₁₀ relative to the solvent weight is generally not less than about 1 w/w%, preferably not less than 3 w/w %, more preferably not less than 10 w/w%, and still more preferably not less than 15 w/w%. The upper limit is not particularly restricted but generally is not higher than about 60 w/w%, preferably not higher than 50 w/w%, more preferably not higher than 40 w/w%, and still more preferably not higher than 30 w/w%. Thus, the reaction can be favorably carried out at a reaction concentration of about 1 to 60 w/w%, preferably about 3 to 50 w/w%, and more preferably about 10 to 40 w/w%.

The reduction reaction time may vary depending on the reducing agent species and/or the amount thereof, hence cannot be absolutely specified. Generally, however, the reaction can be driven to completion within 48 hours, preferably within 24 hours, more preferably within 10 hours, and still more preferably within 5 hours.

After the reduction reaction, an organic phase containing the product reduced coenzyme Q₁₀ or the oily reduced coenzyme Q₁₀ is recovered (e.g. recovered by separation, extraction, concentration, etc.), and if necessary (preferably), the organic phase is further washed repeatedly using water, brine, or the like to achieve contaminant elimination and, then, it can be subjected to crystallization as such or after dissolution to or substitution by other desirable solvents.

As the other solvents, there may be mentioned, for example, hydrocarbons, fatty acid esters, ethers, alcohols, fatty acids, ketones, nitrogen compounds (inclusive of nitriles and amides), sulfur compounds, etc. mentioned above or below.

They were found that, the above-mentioned series of processes from the reduction reaction to the aftertreatment is particularly preferably carried out under a deoxygenated atmosphere, and, in the reduction reaction using dithionous acid or a salt thereof, in particular, such atmosphere greatly contributes to an improvement in reduction reaction yield and a reduction in reducing agent amount. The deoxygenated atmosphere can be attained by substitution with an inert gas, pressure reduction, boiling, or a combination of these. It is preferable to carry out at least the substitution with an inert gas, namely to use an inert gas atmosphere. As the inert gas, there may be mentioned, for example, nitrogen gas, helium gas, argon gas, hydrogen gas, and carbon dioxide gas. Nitrogen gas is preferred, however.

The crystallization of reduced coenzyme Q₁₀ of the invention is now described.

The reduced coenzyme Q₁₀ to be subjected to crystallization can be obtained in the conventional manner, for example, by synthesis, fermentation, or extraction from a natural source. Preferred is the product obtained by reduction of oxidized coenzyme Q₁₀. More preferred is a solution or oil of the reduced coenzyme Q₁₀ obtained by carrying out the reduction reaction in accordance with the present invention, as described above. Still more preferred is the one obtained by reducing the oily oxidized coenzyme Q₁₀ in an aqueous solution using dithionites, or the one obtained by reducing the oxidized coenzyme Q₁₀ using alcohols and/or water-soluble organic solvents.

While the method of crystallization according to the invention can be applied also to products containing oxidized coenzyme Q₁₀ in relatively large amounts, the method is particularly effective in crystallizing high-purity reduced coenzyme Q₁₀ prepared by the reduction method described above.

In the present invention, the reduced coenzyme Q₁₀ is crystallized in an aqueous solution. Use of water contributes to the economical efficiency obviously, and to the safety on industrial operation and product safety as well. Moreover, the use of water also contributes to improvement of the slurry property and the yield of the reduced coenzyme Q₁₀, and can promote isolation of the reduced coenzyme Q₁₀ efficiently by leaving a reducing agent used in the reduction reaction or impurities derived from the reducing agent in a mother liquor. Furthermore, the above-mentioned use of water, or preferably the use of water containing salts can protect the reduced coenzyme Q₁₀ from oxidization by molecular oxygen.

The above salts are not particularly restricted but there may be mentioned, for example, salts constituted from alkaline metals such as lithium, sodium and potassium; alkaline earth metals such as magnesium and calcium with halogen atoms such as fluorine, chlorine and bromine; a residue obtained by removing a proton from inorganic acids such as sulfuric acid and organic acids such as formic acid, acetic acid and propionic acid. Among these, preferred are inorganic salts, and more preferred are sodium chloride, potassium chloride, sodium sulfate, etc.

The concentration of the above salts is preferably high, and it is generally 3 w/w% or more, preferably 5 w/w% or more, more preferably 10 w/w% or more. Still more preferred is that the above salts are dissolved in water at saturation or close to saturation.

As the method of crystallizing the reduced coenzyme Q₁₀ mentioned above, general crystallization methods such as crystallization by cooling, crystallization by concentration and crystallization by solvent substitution can be used. It is preferred to use the crystallization by cooling singly or in combination. As particularly preferable embodiments, the following two methods may be mentioned.
(1) A crystallization method which comprises substituting an organic solvent solution containing the reduced coenzyme Q₁₀ (e.g. reaction solution, extraction solution, etc.) by water (for increasing the composition ratio of water).
(2) A method of crystallizing the oily reduced coenzyme Q₁₀ in an aqueous solution.

In the above-mentioned two methods, it is more preferable to use the crystallization by cooling singly or in combination.

Firstly, the method (1) is explained.

The organic solvent containing the reduced coenzyme Q₁₀ is not particularly restricted but there may be mentioned, for example, hydrocarbons, fatty acid esters, ethers, alcohols, fatty acids, ketones, nitrogen compounds (inclusive of nitriles and amides), sulfur compounds, etc.

As the hydrocarbons, fatty acid esters, ethers and nitriles, those exemplified as the reaction solvent in the fore-mentioned explanation about reduction of the oxidized coenzyme Q₁₀ can be preferably used.

The alcohols are not particularly restricted but may be cyclic or acyclic, or saturated or unsaturated. Saturated ones are preferred, however. Generally, they contain 1 to 20 carbon atoms, preferably 1 to 12 carbon atoms, more preferably 1 to 6 carbon atoms. Still more preferred are monohydric alcohols containing 1 to 5 carbon atoms, dihydric alcohols containing 2 to 5 carbon atoms, and the trihydric alcohol containing 3 carbon atoms.

As the monohydric alcohol, there may be mentioned, for example, methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol, neopentyl alcohol, 1-hexanol, 2-methyl-1-pentanol, 4-methyl-2-pentanol, 2-ethyl-1-butanol, 1-heptanol, 2-heptanol, 3-heptanol, 1-octanol, 2-octanol, 2-ethyl-1-hexanol, 1-nonanol, 1-decanol, 1-undecanol, 1-dodecanol, allyl alcohol, propargyl alcohol, benzyl alcohol, cyclohexanol, 1-methylcyclohexanol, 2-methylcyclohexanol, 3-methylcyclohexanol, 4-methylcyclohexanol, etc.

Preferred are methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol, neopentyl alcohol, 1-hexanol, 2-methyl-1-pentanol, 4-methyl-2-pentanol, 2-ethyl-1-butanol and cyclohexanol.

More preferred are methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol and neopentyl alcohol. Still more preferred are methanol, ethanol, 1-propanol, 2-propanol, 1-butanol, 2-butanol, isobutyl alcohol, 2-methyl-1-butanol and isopentyl alcohol. Most preferred is ethanol.

As the dihydric alcohol, there maybe mentioned, for example, 1,2-ethanediol, 1,2-propandiol, 1,3-propandiol, 1,2-butanediol, 1,3-butanediol, 1,4-butanediol, 2,3-butanediol, 1,5-pentanediol, etc. Preferred are 1,2-ethanediol, 1,2-propandiol and 1,3-propandiol, and most preferred is 1,2-ethanediol.

As the trihydric alcohol, glycerol, etc. may be preferably used, for example.

As fatty acids, there maybe mentioned, for example, formic acid, acetic acid, propionic acid, etc. Preferred are formic acid and acetic acid, and most preferred is acetic acid.

The ketones are not particularly restricted, and ones having 3 to 6 carbon atoms are preferably used in general. As specific examples, there may be mentioned, for example, acetone, methyl ethyl ketone, methyl butyl ketone, methyl isobutyl ketone, etc. Preferred are acetone and methyl ethyl ketone, and most preferred is acetone.

As the nitrogen compounds other than nitriles, there may be mentioned, for example, nitromethane, triethylamine, pyridine, formamide, N-methylformamide, N, N-dimethylformamide, N,N-dimethylacetoamide, N-methylpyrrolidone, etc.

As the sulfur compounds, there may be mentioned, for example, dimethyl sulfoxide, sulfolane, etc.

In selecting the solvent to be used from among the solvents mentioned above, such properties as boiling point and viscosity (for example, the solvent shouldhave a boiling point which allows appropriate warming for increasing the solubility and facilitates a solvent removal from wet masses by drying and solvent recovery from crystallization filtrates (about 30 to 150°C at 1 atm), a melting point such that solidification hardly occurs in handling at room temperature as well as upon cooling to room temperature or below (not higher than about 20°C, preferably not higher than about 10°C, still more preferably not higher than about 0°C), and a low viscosity (not higher than about 10 cp at 20°C)) are preferably taken into consideration. From the industrial operation viewpoint, a solvent which is scarcely volatile at ordinary temperature is preferred.

Among the above solvents, in particular, hydrocarbons, fatty acid esters, ethers and nitriles can be preferably used in view of the oxidization protection of the reduced coenzyme Q₁₀ mentioned above. Additionally, in view of obtaining the high yield while preferably decreasing the solubility of the reduced coenzyme Q₁₀, alcohols, fatty acids, ethers, ketones and nitriles can be preferably used. From a viewpoint of an industrial applicability, for example, hydrocarbons and alcohols can be preferably used.

In the above method (1), as a means for substituting the organic solvent solution containing the reduced coenzyme Q₁₀ by water, there may be mentioned, for example, a method comprising increasing the water ratio while concentrating and removing the organic solvent. Furthermore, if necessary, operations such as cooling and seed crystal addition, as to be described below, can be combinedly used properly.

Next, the method (2), i.e., the method of crystallizing the oily reduced coenzyme Q₁₀ in an aqueous solution is explained. According to this method, the reduced coenzyme Q₁₀ crystal having a large particle diameter can be obtained, and filterability can be remarkably improved.

The crystallization may be carried out by adding water to the oily reduced coenzyme Q₁₀, for example, or on the contrary, by adding the oily reduced coenzyme Q₁₀ to water. Moreover, the crystallization may be also carried out by cooling a mixture of the oily reduced coenzyme Q₁₀ and water. More preferred is a method comprising removing an organic solvent from a mixed solvent solution consisting of an organic solvent containing the reduced coenzyme Q₁₀ and water at a temperature not lower than the melting point of the reduced coenzyme Q₁₀ or of a concentrate comprising the reduced coenzyme Q₁₀ as a main component, to obtain oil in the system, and cooling to crystallize the oil.

The above-mentioned temperature not lower than the melting point is generally 45°C or more, preferably 48°C or more, and more preferably 50°C or more although it is dependent on the purity, etc. of the reduced coenzyme Q₁₀. The upper limit is not particularly restricted but generally 100°C or less is preferred, 80°C or less is more preferred, and 60°C or less is still more preferred.

In the crystallization method of the present invention as described above, the crystallization temperature of the reduced coenzyme Q₁₀ (cooling temperature at the time of crystallization) is generally 48°C or less, preferably 45°C or less, more preferably 40°C or less, and still more preferably 30°C or less in view of the yield, etc., although it is difficult to uniformly define since it is dependent on the purity of the reduced coenzyme Q₁₀. The lower limit is a solidification temperature of the system. Therefore, the crystallization can be especially preferably carried out at the crystallization temperature of about 0 to 30°C.

It is preferable to control the amount of crystallization perunit time in crystallizing, i.e. the rate of crystallization. The preferable amount of crystallization per unit time is, for example, not higher than the rate of crystallization which causes crystallization of about 50%, per unit time, of the whole amount of crystals to be obtained (i.e. at most 50%/hour), preferably not higher than the rate of crystallization which causes crystallization of about 25%, per unit time, of the whole amount of crystals to be obtained (i.e. at most 25%/hour).

The rate of cooling in the crystallization by cooling is generally not higher than about 40°C/hour, and preferably not higher than about 20°C/hour.

The crystallization is preferably carried out under forced flowing in order to prevent the state of supersaturation from occurring and thereby allowing the nucleation and crystal growth to proceed smoothly, in order to obtain crystals with uniform particle diamater and, furthermore, from the viewpoint of obtaining high-quality products. The flowing is generally brought about by a stirring power per unit volume of not weaker than about 0.01 kW/m³, preferably not weaker than about 0.1 kW/m³, and more preferably not weaker than about 0.3 kW/m³. The forced flowing is generally provided by the turning of a stirring blade(s). However, the use of a stirring blade (s) is not always necessary if the above flowing can be otherwise obtained. For example, it is possible to utilize a method based on liquid circulation.

In carrying out the crystallization, seed crystals are preferably added so that the state of supersaturation may be prevented from occurring and the nucleation and crystal growth may be allowed to proceed smoothly.

The crystallization concentration, when expressed in terms of the weight of reduced coenzyme Q₁₀ relative to the total weight of the crystallization solvent at the time of completion of crystallization, it is not higher than about 15 w/w%, preferably not higher than about 13 w/w%, more preferably not higher than 10 w/w%. From the productivity viewpoint, the lower limit to the crystallization concentration is generally not lower than about 1 w/w%, preferably not lower than about 2 w/w%.

The thus-obtained crystals of reduced coenzyme Q₁₀ can be recovered as a wet product, for example, by such a solid-liquid separation technique as centrifugation, pressure filtration, or vacuum filtration, if necessary followed by cake washing. They can be recovered also as a dry product by further charging the wet product in a reduced pressure drier (vacuum drier) internally purged with an inert gas and drying the same under reduced pressure. The recovery in a dry form is preferred.

The crystallization of the invention, when it is carried out in a deoxygenated atmosphere, can increase protective effect against oxidation. The deoxygenated atmosphere can be attained by inert gas substitution, pressure reduction, boiling, or a combination of these. It is preferable to carry out at least the substitution with an inert gas, namely to use an inert gas atmosphere. As the inert gas, there may be mentioned, for example, nitrogen gas, helium gas, argon gas, hydrogen gas, and carbon dioxide gas. Nitrogen gas is preferred, however.

In accordance with the present invention, high-quality reduced coenzyme Q₁₀ can be obtained with excellent workability and economical efficiency. The crystals of reduced coenzyme Q₁₀ as obtained in accordance with the present invention are of very high quality and can be expected to have a reduced coenzyme Q₁₀/oxidized coenzyme Q₁₀ weight ratio of not lower than 96/4, preferably not lower than 98/2, more preferably not lower than 99/1.

### BEST MODE FOR CARRYING OUT THE INVENTION

The following examples illustrate the present invention in further detail. These examples are, however, by no means limitativeof the scope of the present invention. In the examples, the purity of reduced coenzyme Q₁₀ and the reduced coenzyme Q₁₀/oxidized coenzyme Q₁₀ weight ratio were determined by the HPLC analysis specified below. The reduced coenzyme Q₁₀ purity values as determined, however, are by no means indicative of the limit purity value attainable in accordance with the present invention. Likewise, the reduced coenzyme Q₁₀/oxidized coenzyme Q₁₀ weight ratio values obtained never indicate the upper limit to that ratio.

### (HPLC conditions)

Column: SYMMETRY C18 (product of Waters), 250 mm (in length), 4.6 mm (in inside diameter); mobile phase: C₂H₅OH:CH₃OH = 4:3 (v/v); detection wavelength: 210 nm; flow rate: 1 ml/min; retention time of reduced coenzyme Q₁₀: 9.1 min; retention time of oxidized coenzyme Q₁₀: 13.3 min.

### (Example 1)

Oxidized coenzyme Q₁₀ (100 g; purity 99.4%) was melted with stirring at 48°C. While stirring (power required for stirring: 0.3 kW/m³), an aqueous solution prepared by dissolving 100 g of sodium dithionites (purity: at least 75%), as the reducing agent, in 1000 ml of water was gradually added to the oil and the reduction reaction was carried out at 48°C and at pH 4 to 6. The aqueous phase was removed from the reaction mixture containing the oil, and the oil was washed 6 times with 1000 g of deaerated saturated brine at 48°C. After that, by removing the aqueous phase, oily reduced coenzyme Q₁₀ was obtained. 1500 g of deaerated water at 48°C was added to this oil, and while stirring (power required for stirring: 0.3 kW/m³), the mixture was cooled to 2°C to obtain white slurry (fluidity of the slurry was good). All the above operations were carried out under a nitrogen atmosphere. The obtained slurry was filtered under reduced pressure, the wet crystal was washed with cold ethanol, cold water and cold ethanol in this order (the temperature of the cold solvent used for washing was 2°C), and further the wet crystal was dried under reduced pressure (20 to 40°C, 1 to 30 mmHg), 97 g of dry white crystal was obtained (isolated product yield: 97 mol %). The weight ratio of the reduced coenzyme Q₁₀/oxidized coenzyme Q₁₀ of the obtained crystal was 99.4/0. 6, and the purity of the reduced coenzyme Q₁₀ was 99.2%.

### (Example 2)

The oxidized coenzyme Q₁₀ (100 g; purity 99.4%) was dissolved in 1000 g of heptane at 25°C. While stirring (power required for stirring: 0.3 kW/m³), an aqueous solution dissolving 100 g of sodium dithionites (purity 75% or more), as a reducing agent, in 1000 ml of water was gradually added and a reduction reaction was carried out at 25°C and at pH 4 to 6. 2 hours later, an aqueous phase was removed from the reaction solution, and a heptane phase was washed 6 times with 1000 g of deaerated saturated brine. 1000 g of water was added to this heptane phase, and while stirring (power required for stirring: 0.3 kW/m³), heptane was removed by reducing pressure at 30°C to obtain white slurry. This slurry had good fluidity and was capable to be easily brushed away from the crystallization container. All the above operations were carried out under a nitrogen atmosphere. The obtained slurry was filtered under reduced pressure, and the wet crystal was washed with cold ethanol, cold water and cold ethanol in this order (the temperature of the cold solvent used for washing was 2°C). The wet crystal was further dried under reduced pressure (20 to 40°C, 1 to 30 mmHg), and a dry white crystal was obtained (isolated product yield: 97 mol %). The weight ratio of reduced coenzyme Q₁₀/oxidized coenzyme Q₁₀ of the obtained crystal was 99.5 / 0.5, and the purity of the reduced coenzyme Q₁₀ was 99.2%.

### (Example 3)

To 1000 g of ethanol, 100 g of the oxidized coenzyme Q₁₀ (purity 99.4%), 60 g of L-ascorbic acid and 30 g of sodium hydrogencarbonate were added, and the mixture was stirred at 78°C and subjected to a reduction reaction. 3 hours later, the mixture was cooled to 50°C, and 1000 g of heptane and 1000 g of deaerated water were added while holding said temperature. After the mixture was cooled to 25°C, an aqueous phase was removed, and further washed 6 times with 1000 g of saturatedbrine to remove the aqueous phase. Heptane was removed from this heptane solution at 48°C to obtain the oily reduced coenzyme Q₁₀. To this oil, 1500 g of deaerated water at 48°C was added, and while stirring (power required for stirring: 0.3 kW/m³), the mixture was cooled to 2°C to obtain white slurry (fluidity of the slurry was good as in Example 1). All the above operations were carried out under the nitrogen atmosphere. The obtained slurry was filtered under reduced pressure, and the wet crystal was washed with cold ethanol, cold water and cold ethanol in this order (the temperature of the cold solvent used for washing was 2°C). The wet crystal was further dried under reduced pressure (20 to 40°C, 1 to 30 mmHg) to obtain 97 g of dry white crystal (isolated product yield: 97 mol%). The weight ratio of the reduced coenzyme Q₁₀/oxidized coenzyme Q₁₀ of the obtained crystal was 99.4/0.6, and the purity of the reduced coenzyme Q₁₀ was 99.2%.

### (Comparative Example 1)

A heptane phase of the reduced coenzyme Q₁₀ after being washed with deaerated saturated brine was obtained in the same manner as Example 2. While stirring this heptane phase (power required for stirring: 0.3 kW/m³), the mixture was cooled to 2°C to obtain white slurry. This slurry was poor in fluidity, and more difficultly brushed away from the crystallization container as compared with Example 1. All the above operations were carried out under the nitrogen atmosphere. The obtained slurry was filtered under reduced pressure, the wet crystal was washed with cold ethanol, cold water and cold ethanol in this order (the temperature of the cold solvent used for washing was 2°C). The wet crystal was further dried under reduced pressure (20 to 40°C, 1 to 30 mmHg) to obtain 93 g of dry white crystal (isolated product yield: 93 mol %). The weight ratio of the reduced coenzyme Q₁₀/oxidized coenzyme Q₁₀ of the obtained crystal was 99.6/0.4, and the purity of the reduced coenzyme Q₁₀ was 99.3%.

### (Reference Example 1)

One gram of the reduced coenzyme Q₁₀ (the weight ratio of the reduced coenzyme Q₁₀/oxidized coenzyme Q₁₀ is 99.6/0.4) was dissolved in 20 g of various solvents shown in Table 1 at 25°C. In the atmosphere, the weight ratio of the reduced coenzyme Q₁₀/oxidized coenzyme Q₁₀ in the solution after stirring for 24 hours at 25°C, and the result is shown in Table 1.

**Table 1**

| **Solvent** | R |
|---|---|
| **Heptane** | 99.1/0.9 |
| **Hexane** | 98.7/1.3 |
| **Toluene** | 98.8/1.2 |
| **Chloroform** | 98.9/1.1 |
| **Ethyl acetate** | 98.9/1.1 |
| **Methyl tert-butyl ether** | 98.6/1.4 |
| **Tetrahydrofuran** | 98.5/1.5 |

| | |
|---|---|
| **R: Reduced coenzyme Q₁₀/oxidized coenzyme Q₁₀ weight ratio** | |

### (Reference Example 2)

One gram of the reduced coenzyme Q₁₀ (the weight ratio of the reduced coenzyme Q₁₀/oxidized coenzyme Q₁₀ is 99.6/0.4) was dissolved in 100 g of various solvents shown in Table 2 at 35°C. In the atmosphere, the weight ratio of the reduced coenzyme Q₁₀/oxidized coenzyme R₁₀ in the solution after stirring for 24 hours at 35°C was measured, and the result is shown in Table 2.

**Table 2**

| **Solvent** | R |
|---|---|
| **Heptane** | 96.7/3.3 |
| **Ethyl acetate** | 96.4/3.6 |
| **Acetonitrile** | 96.0/4.0 |

| | |
|---|---|
| **R: Reduced coenzyme Q₁₀/oxidized coenzyme Q₁₀weight ratio** | |

### INDUSTRIAL APPLICABILITY

The invention, which has the constitution described above, is a method excellent in workability and economical efficiency on the industrial scale and can give high-quality reduced coenzyme Q₁₀ in a convenient and efficient manner.

## Claims

1. A method of crystallizing a reduced coenzyme Q₁₀
which comprises crystallizing the reduced coenzyme Q₁₀ in an aqueous solution.

2. The method according to Claim 1,
wherein the reduced coenzyme Q₁₀ is crystallized by substituting an organic solvent containing the reduced coenzyme Q₁₀ by water.

3. The method according to Claim 1,
wherein oily reduced coenzyme Q₁₀ is crystallized in an aqueous solution.

4. The method according to Claim 3,
wherein the crystallization is carried out by cooling a mixture of the oily reduced coenzyme Q₁₀ and water.

5. The method according to Claim 4,
wherein an organic solvent is removed from a solution of a mixed solvent comprising an organic solvent containing the reduced coenzyme Q₁₀ and water at a temperature not lower than the melting point of the reduced coenzyme Q₁₀ or of a concentrate comprising the reduced coenzyme Q₁₀ as a main component, to obtain oily reduced coenzyme Q₁₀ in the system, and cooled to crystallize the oily reduced coenzyme Q₁₀.

6. The method according to any one of Claims 1 to 5
wherein the crystallization by cooling is used singly or in combination.

7. The method according to any one of Claims 1 to 6,
wherein the crystallization temperature is 48°C or less.

8. The method according to any one of Claims 1 to 7,
wherein the crystallization is carried out under forced flowing with a stirring power per unit volume of not weaker than 0.01 kW/m³.

9. The method according to any one of Claims 1 to 8,
wherein a seed crystal is added in the crystallization.

10. The method according to any one of Claims 1 to 9,
in which the rate of crystallization is not higher than the rate of crystallization which causes crystallization of 50%, per unit time, of the whole amount of crystals.

11. The method according to any one of Claims 1 to 10,
wherein the crystallization is carried out under a deoxygenated atmosphere.

12. The method according to any one of Claims 1 to 11,
wherein the reduced coenzyme Q₁₀ used for the crystallization is obtained by reducing an oxidized coenzyme Q₁₀.

13. The method according to any one of Claims 1 to 12,
wherein the reduced coenzyme Q₁₀ used for the crystallization is obtained by reducing oily oxidized coenzyme Q₁₀ in an aqueous solution using dithionites.

14. The method according to any one of Claims 1 to 12,
wherein the reduced coenzyme Q₁₀ used for the crystallization is obtained by reducing the oxidized coenzyme Q₁₀ using alcohols and/or water-soluble organic solvents.

15. The method according to any one of Claims 1 to 14,
wherein a reducing agent used in the reduction reaction or impurities derived from the reducing agent is removed to a mother liquor in crystallizing the reduced coenzyme Q₁₀.

## Patentansprüche

1. Verfahren zur Kristallisierung eines reduzierten
Coenzyms Q₁₀, das die Kristallisierung des reduzierten Coenzyms Q₁₀ in wäßriger Lösung umfaßt.

2. Verfahren gemäß Anspruch 1,
wobei das reduzierte Coenzym Q₁₀ durch Austausch eines organischen Lösungsmittels, das das reduzierte Coenzym Q₁₀ enthält, durch Wasser kristallisiert wird.

3. Verfahren gemäß Anspruch 1,
wobei das ölige reduzierte Coenzym Q₁₀ in einer wäßrigen Lösung kristallisiert wird.

4. Verfahren gemäß Anspruch 3,
wobei die Kristallisierung mittels Abkühlens einer Mischung des öligen reduzierten Coenzyms Q₁₀ und Wasser durchgeführt wird.

5. Verfahren gemäß Anspruch 4,
wobei ein organisches Lösungsmittel aus einer Lösung eines gemischten Lösungsmittels, umfassend ein organisches Lösungsmittel, das das reduzierte Coenzym Q₁₀ enthält, und Wasser bei einer Temperatur, die nicht niedriger ist als die Schmelztemperatur des reduzierten Coenzyms Q₁₀ oder eines Konzentrats, umfassend das reduzierte Coenzym Q₁₀ als Hauptkomponente, abgetrennt wird, um das ölige reduzierte Coenzym Q₁₀ im System zu erhalten, und mittels Abkühlung das ölige reduzierte Coenzym Q₁₀ kristallisiert wird.

6. Verfahren gemäß einem der Ansprüche 1 bis 5,
wobei die Kristallisierung mittels Abkühlung einzeln oder in Kombination angewendet wird.

7. Verfahren gemäß einem der Ansprüche 1 bis 6,
wobei die Kristallisierungstemperatur 48°C oder niedriger ist.

8. Verfahren gemäß einem der Ansprüche 1 bis 7,
wobei die Kristallisierung unter Zwangsdurchströmung bei einer Rührleistung pro Volumeneinheit von nicht weniger als 0,01 kW/m³ durchgeführt wird.

9. Verfahren gemäß einem der Ansprüche 1 bis 8,
wobei ein Impfkristall bei der Kristallisierung beigefügt wird.

10. Verfahren gemäß einem der Ansprüche 1 bis 9,
wobei die Kristallisierungsgeschwindigkeit nicht höher ist als die Kristallisierungsgeschwindigkeit, die eine Kristallisierung von 50 % der Gesamtmenge der Kristalle pro Zeiteinheit hervorruft.

11. Verfahren gemäß einem der Ansprüche 1 bis 10, wobei die Kristallisierung unter einer deoxygenierten Atmosphäre durchgeführt wird.

12. Verfahren gemäß einem der Ansprüche 1 bis 11,
wobei das für die Kristallisierung verwendete reduzierte Coenzym Q₁₀ durch Reduktion von oxidiertem Coenzym Q₁₀ erhalten wird.

13. Verfahren gemäß einem der Ansprüche 1 bis 12,
wobei das für die Kristallisierung verwendete reduzierte Coenzym Q₁₀ durch Reduktion von öligem oxidiertem Coenzym Q₁₀ in einer wäßrigen Lösung unter Verwendung von Dithioniten erhalten wird.

14. Verfahren gemäß einem der Ansprüche 1 bis 12,
wobei das für die Kristallisierung verwendete reduzierte Coenzym Q₁₀ durch Reduktion von oxidiertem Coenzym Q₁₀ unter Verwendung von Alkoholen und/oder wasserlöslichen organischen Lösungsmitteln erhalten wird.

15. Verfahren gemäß einem der Ansprüche 1 bis 14,
wobei ein in der Reduktionsreaktion verwendetes Reduktionsmittel oder vom Reduktionsmittel stammende Verunreinigungen während der Kristallisierung des reduzierten Coenzyms Q₁₀ in eine Mutterlauge entfernt werden.

## Revendications

1. Procédé de cristallisation d'une coenzyme Q₁₀ réduite, qui comprend la cristallisation de la coenzyme Q₁₀ réduite dans une solution aqueuse.

2. Procédé selon la revendication 1, dans lequel on cristallise la coenzyme Q₁₀ réduite en remplaçant un solvant organique contenant la coenzyme Q₁₀ réduite par de l'eau.

3. Procédé selon la revendication 1, dans lequel on cristallise une coenzyme Q₁₀ réduite huileuse dans une solution aqueuse.

4. Procédé selon la revendication 3, dans lequel on effectue la cristallisation en refroidissant un mélange de la coenzyme Q₁₀ réduite huileuse et d'eau.

5. Procédé selon la revendication 4, dans lequel on sépare un solvant organique d'une solution d'un solvant mixte comprenant un solvant organique contenant la coenzyme Q₁₀ réduite et de l'eau à une température qui n'est pas inférieure au point de fusion de la coenzyme Q₁₀ réduite ou d'un concentré comprenant la coenzyme Q₁₀ réduite comme constituant principal, pour obtenir de la coenzyme Q₁₀ réduite huileuse dans le système, et on refroidit pour cristalliser la coenzyme Q₁₀ réduite huileuse.

6. Procédé selon l'une quelconque des revendications 1 à 5, dans lequel on utilise la cristallisation par refroidissement isolément ou en combinaison.

7. Procédé selon l'une quelconque des revendications 1 à 6, dans lequel la température de cristallisation est d'au plus 48°C.

8. Procédé selon l'une quelconque des revendications 1 à 7, dans lequel la cristallisation s'effectue dans des conditions d'écoulement forcé avec une puissance d'agitation par unité de volume d'au moins 0,01 kW/m³.

9. Procédé selon l'une quelconque des revendications 1 à 8, dans lequel on ajoute un germe cristallin dans la cristallisation.

10. Procédé selon l'une quelconque des revendications 1 à 9, dans lequel la vitesse de cristallisation n'est pas supérieure à la vitesse de cristallisation qui entraîne la cristallisation de 50 % de la quantité totale de cristaux par unité de temps.

11. Procédé selon l'une quelconque des revendications 1 à 10, dans lequel la cristallisation s'effectue sous une atmosphère désoxygénée.

12. Procédé selon l'une quelconque des revendications 1 à 11, dans lequel la coenzyme Q₁₀ réduite utilisée pour la cristallisation est obtenue par réduction d'une coenzyme Q₁₀ oxydée.

13. Procédé selon l'une quelconque des revendications 1 à 12, dans lequel la coenzyme Q₁₀ réduite utilisée pour la cristallisation est obtenue par réduction d'une coenzyme Q₁₀ oxydée huileuse dans une solution aqueuse à l'aide de dithionites.

14. Procédé selon l'une quelconque des revendications 1 à 12, dans lequel la coenzyme Q₁₀ réduite utilisée pour la cristallisation est obtenue par réduction de la coenzyme Q₁₀ oxydée à l'aide d'alcools et/ou de solvants organiques solubles dans l'eau.

15. Procédé selon l'une quelconque des revendications 1 à 14, dans lequel un agent réducteur utilisé dans la réaction de réduction ou des impuretés dérivées de l'agent réducteur est transféré dans une liqueur mère en cristallisant la coenzyme Q₁₀ réduite.
